Europäisches Patentamt

(19) European Patent Office

Office européen des brevets

(11) EP 0 397 056 B1

(12) EUROPEAN PATENT SPECIFICATION

(45) Date of publication and mention
of the grant of the patent:
04.09.1996 Bulletin 1996/36

(51) Int. Cl.$^6$: **A61B 17/225**, A61H 23/00

(21) Application number: 90108462.4

(22) Date of filing: 04.05.1990

(54) **Shock wave generating apparatus**

Gerät zur Erzeugung von Stosswellen

Appareil de génération d'ondes de choc

(84) Designated Contracting States:
**DE FR**

(30) Priority: 08.05.1989 JP 113767/89

(43) Date of publication of application:
14.11.1990 Bulletin 1990/46

(73) Proprietor: **KABUSHIKI KAISHA TOSHIBA**
**Kawasaki-shi, Kanagawa-ken 210 (JP)**

(72) Inventors:
• **Kudo, Nobuki**
**Nasu-gun, Tochigi-ken (JP)**
• **Terai, Fujio**
**Yokohama-shi, Kanagawa-ken (JP)**

(74) Representative: **Blumbach, Kramer & Partner**
**Patentanwälte**
**Radeckestrasse 43**
**81245 München (DE)**

(56) References cited:
**EP-A- 0 169 311** **EP-A- 0 225 104**
**EP-A- 0 269 801** **EP-A- 0 301 360**
**DE-A- 3 721 187** **DE-A- 3 826 709**
**US-A- 4 763 652** **US-A- 4 821 729**

• **Biliary Lithotripsy (Eds. J.T. Ferrucci et al.),**
**Adapted from the Proceedings of the First**
**International Symposium on Biliary Lithotripsy,**
**July 11-13, 1988, pages 253-263, 263a; B.**
**Forssman et al.: "Dornier: MPL 9000**
**Multipurpose Lithotripter"**

Printed by Rank Xerox (UK) Business Services
2.13.4/3.4

## Description

### Background of the Invention

### Field of the Invention

The present invention generally relates to a shock wave generating apparatus capable of disintegrating an article, e.g., cancer cell, and a concretion within a biological object under medical examination, by utilizing focused energy of a shock waves. More specifically, the present invention is directed to a shock wave generating apparatus capable of setting a moving direction of a shock wave generating source to an ultrasonic tomographic image plane formed by an ultrasonic probe.

### Description of the Related Art

Various types of shock wave generating apparatuses have been proposed in, for instance, Japanese KOKAI (Disclosure) patent application No. 62-49843 (1987).

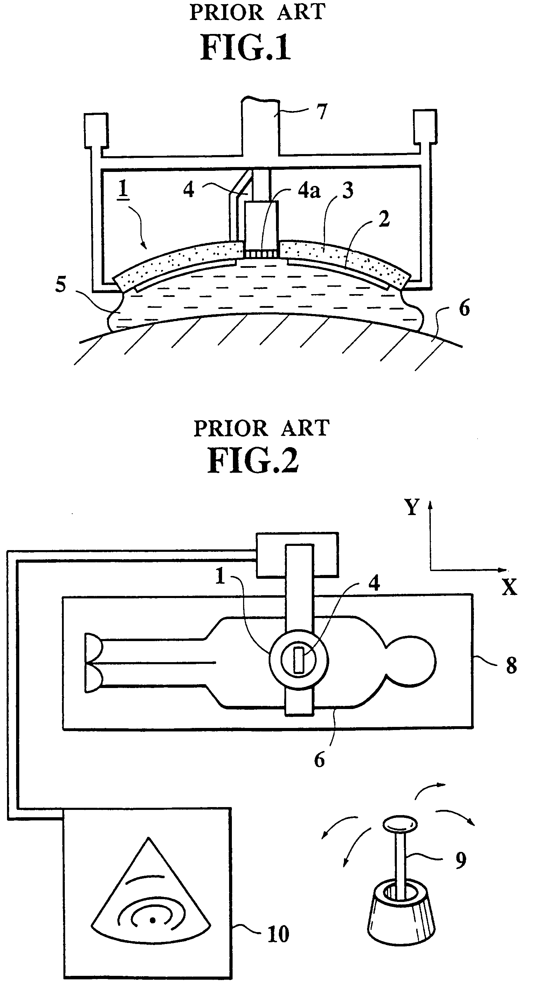

In Fig. 1, there is shown, as a sectional view, an ultrasonic wave applicator 1 of one conventional shock wave generating apparatus.

The construction of this ultrasonic wave applicator 1 is as follows. A through hole having a predetermined shape is formed in a center portion of this applicator 1. A vibrating element (e.g., piezoelectric transducer element) 2 is spherically formed and a backing material 3 is uniformly adhered to a rear surface of this spherical vibrating element 2. An imaging ultrasonic probe 4 is positioned in such a manner that a transmitting/receiving wave front (ultrasonic array) 4a is located at the curved surface identical to the shock wave transmitting/receiving wave front of the vibrating element 2, or backward the last-mentioned wave front. Furthermore, this ultrasonic wave applicator 1 includes a water bag 5 containing water as a coupling medium for the ultrasonic wave. Reference numeral 6 indicates a biological body under medical examination.

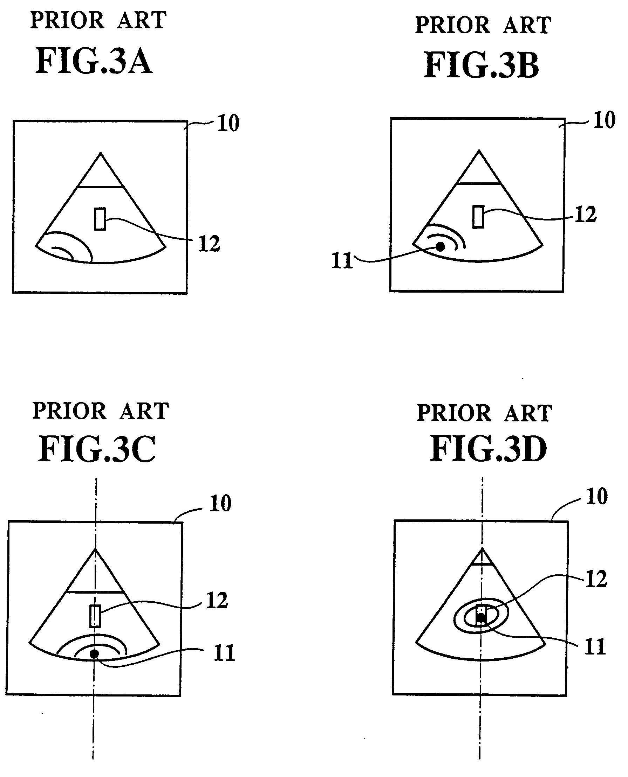

Referring now to Figs. 2 and 3A to 3D, a description will be made to a means for identifying a position of an article to be disintegrated, e.g., a concretion or calculus positioned within the biological body 6 with employment of the above-described conventional ultrasonic applicator 1 shown in Fig. 1. That is to say, this implies a means for discovering the actual position of the concretion within the biological body 6 and for making the focal point of the shock wave coincident with the actual concretion position. As shown in Fig. 2, the conventional ultrasonic applicator 1 supported on a supporting member 7 is moved within an X-Y plane over the biological body 6 mounted on a couch 8. This moving operation is controlled by a joy stick 9. A sector-formed ultrasonic beam is projected from the ultrasonic probe 4 employed within the applicator 1, so that a B-mode ultrasonic image (i.e., slice image) of the biological body 6 is formed in the known ultrasonic image forming method and then displayed on a television (TV) monitor 10.

An operator manipulates a joy stick 9 while observing the CT image displayed on the TV monitor 10. For instance, Fig. 3A represents a screen of the TV monitor 10, on which no concretion to be disintegrated within the biological body 6 is discovered, or appears. When the ultrasonic probe 4 is moved by the operator and the concretion is captured by the ultrasonic probe 4, an image 11 of the concretion is displayed on the TV monitor 10 as shown in Fig. 3B. Subsequently, as represented in Fig. 3C, the ultrasonic applicator 1 is moved in such a manner that the image 11 of the concretion to be disintegrated is positioned at a center of the screen on which a marker 12 to indicate a focal point of a shock wave is also displayed. Then, as shown in Fig. 3D, the ultrasonic applicator 1 is moved downwardly in such a way that the concretion image 11 is moved toward a higher direction of the TV monitor 10 so as to be coincident with the position of the focal point marker 12. As a result, the position of the concretion within the biological body 6 may be identified by the focal point of the shock wave.

As apparent from the foregoings, since the conventional means for identifying the article to be disintegrated is used for the ultrasonic diagnostic apparatus as an imaging means of a tissue within a biological body, such an ultrasonic identifying means has a particular advantage that, for instance, a gallstone mainly containing cholesterol, and a soft tissue can be visualized, as compared with the use of the X-ray diagnostic apparatus. However, the ultrasonic diagnostic apparatus functioning as the concretion identifying means has the following drawbacks. That is, since generally, an ultrasonic diagnostic apparatus is to obtain a two-dimensional CT image of a biological body in parallel to an array direction of transducer elements just under an ultrasonic probe, it is rather difficult to identify a position of an article to be disintegrated, as compared with an X-ray diagnostic apparatus for convoluting three-dimensional images along a depth direction of the biological body so as to acquire a two-dimensional image.

That is to say, an article to be disintegrated is continuously displayed on a TV monitor of an X-ray diagnostic ((fluoroscopic)) apparatus when this article is present within a field of view, whereas an article to be disintegrated is displayed on a TV monitor of an ultrasonic diagnostic apparatus only when this article is positioned on a tomographic image plane (slice plane) produced by way of an ultrasonic probe. As a consequence, in case that an article to be disintegrated is discovered from a slice plane produced by way of an ultrasonic probe by an operator and then an image of this article is coincidence with a focal point marker of a disintegrating shock wave displayed on a monitor screen, this article's image may disappear from the monitor screen of the ultrasonic diagnostic apparatus unless a source of an ultrasonic shock wave is moved along a direction coincident with the above-described slice plane. Under these circumstances, the conventional identifying operation for the position of the article to be disintegrated becomes very difficult, because the shock wave generating source must be transported in such a manner that the article to be disintegrated never disappear from the ultrasonic slice image produced by way of the ultrasonic probe.

In Biliary Lithotripsy (Eds. J.T. Ferrucci et al.), Adapted from the Proceedings of the First International Symposium on Biliary Lithotripsy, July 11-13, 1988, pages 253-263, 263a; B. Forssman et al.: "Dornier: MPL 9000 Multipurpose Lithotripter" there is disclosed a shock wave generating apparatus including the features of the preamble of the appended main claim. This known shock wave generating apparatus uses two ultrasonic probe means. One "out-line" transducer is used for surveilling e.g. a gallbladder and a gallstone to be disintegrated. After identifying a gallstone the patient is automatically moved into the treatment position based on computer calculations. The second transducer, the "in-line" transducer is used to monitor the stone-disintegration process in real time. This second transducer can also be used to retarget the stone if the patient moves. The shock wave generating apparatus is further equipped with a remote control unit with buttons for operating the apparatus in different modes. These modes are positioning a patient within a scan plane, vertical to the scan plane, manual patient positioning in longitudinal, lateral, vertical directions or rotation around the longitudinal axis, and automatic patient positioning. This known shock wave generating apparatus has an improved operational comfort in comparison to the shock wave generating apparatuses as mentioned above. But by using two different ultrasonic probe means, a transporting means for moving the complete bed with the patient lying on it and sophisticated automatic computer control circuitry this apparatus is expensive.

The present invention has been made in an attempt to solve the above-described problems and therefore has an object to provide a shock wave generating apparatus capable of simply identifying a position of an article to be disintegrated on a ultrasonic slice plane displayed on a monitor screen. More specifically, another object of the present invention is to provide such a shock wave generating apparatus that when a focal point of a shock wave generating source is made coincident with an image of an article to be disintegrated on a monitor screen of an ultrasonic diagnostic apparatus after the image of this article has been displayed on this monitor screen, a moving (transport) direction of the generating source can be automatically set to the tomographic image plane of the ultrasonic probe.

Summary of the Invention

To achieve these objects a shock wave generating apparatus according to the present invention comprises source means for producing a shock wave and transmitting said shock wave to a biological body under medical examination; ultrasonic imaging means including ultrasonic probe means to project an ultrasonic wave beam to said biological body for producing and displaying an ultrasonic tomographic image of tissue within said biological body; probe position detecting means for detecting a position of said ultrasonic probe means so as to produce a positioning signal when an image of an article to be disintegrated located within said tissue of said biological body is being displayed in said ultrasonic tomographic image; transporting means for changing the relative position between said article to be disintegrated and said source means which transporting means transports both said source means and said ultrasonic probe means; transport controlling means for controlling the transport means in response to the position signal as to position the article to be disintegrated to coincide with the focal point of said shock wave produced by said source means, and generating means provided for generating a marker in said ultrasonic tomographic image indicative of the focal point of said shock wave produced by said source means; which shock wave generating apparatus is characterized in that said probe position detecting means detects a slice plane position of said ultrasonic probe means the axis of which is adapted to rotate so that the ultrasonic tomographic image can be viewed along a rotable slice plane; and the transporting controlling means controls the transport means so as to transport both said source means and said ultrasonic probe means along the detected slice plane in response to the slice plane positioning signal, whereby said marker is coincident with said image of said article to be disintegrated.

The appended subclaims are directed towards advantageous embodiments of the inventive shock wave generating apparatus.

Brief Description of the Drawings:

For a better understanding of the present invention, reference is made to the following description in conjunction with the accompanying drawings, in which:

Fig. 1 is a sectional view of an ultrasonic pulse applicator employed in a conventional shock wave generating apparatus;
Fig. 2 is an illustration for schematically explaining operations of the conventional shock wave generating apparatus employing the ultrasonic pulse application shown in Fig. 1;

Fig. 3A to 3D are illustrations for explaining a conventional identifying operation of an article to be disintegrated on an ultrasonic tomographic image;

Fig. 4 is a schematic block diagram of an arrangement of a shock wave generating apparatus 100 according to a first preferred embodiment of the present invention;

Fig. 5A and 5B are illustrations for schematically representing a probe coordinate system and a coordinate conversion between the ultrasonic coordinate system and an absolute coordinate system;

Fig. 6 is a perspective view of the first shock wave generating apparatus 100 shown in Fig. 4; and,

Fig. 7 is a schematic block diagram of internal circuits of the major arrangements 34, 35, 37 employed in the first shock wave generating apparatus 100 shown in Fig. 1.

Detailed Description of the Preferred Embodiments

OVERALL ARRANGEMENT OF FIRST SHOCK WAVE GENERATING APPARATUS

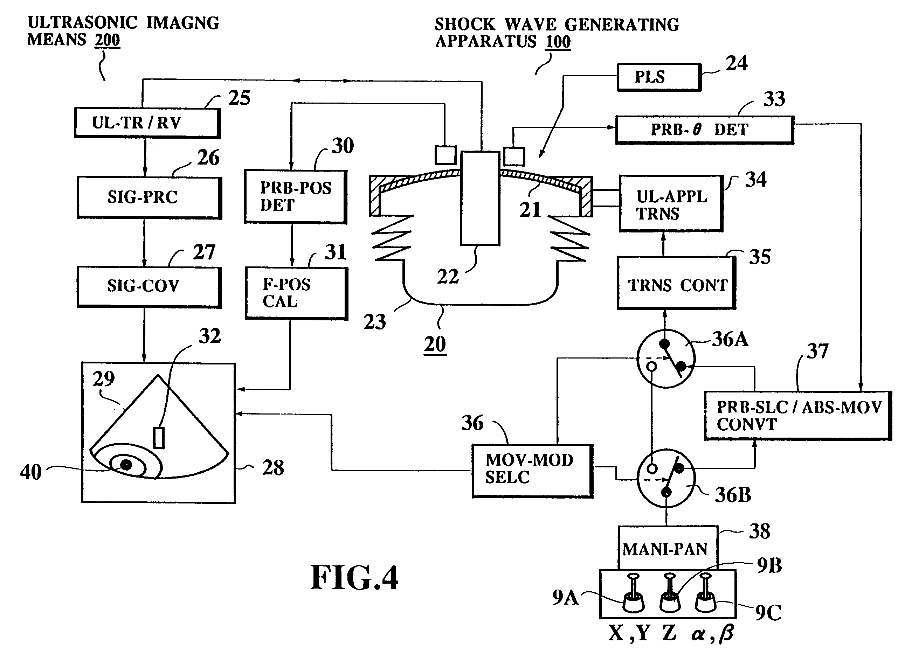

Referring now to Fig. 4, an overall arrangement of a shock wave generating apparatus 100 according to a first preferred embodiment of the present invention, in conjunction with an ultrasonic imaging apparatus 200, will be described.

As apparent from the arrangement shown in Fig. 4, the first shock wave generating apparatus 100 utilizes an ultrasonic wave as a shock wave.

An ultrasonic (wave) applicator 20 is employed in the first shock wave generating apparatus 100 and is so designed to be freely transported in various directions along a biological body (not shown in detail) under medical examination by a transport mechanism (will be discussed later). A shock wave transducer 21 having a concave surface, as viewed in a sectional plane thereof, is provided on a rear surface of this ultrasonic applicator 20 so as to produce therefrom an ultrasonic wave as a shock wave. Furthermore, an ultrasonic (wave) probe 22 of an ultrasonic imaging apparatus 200 is provided at a central portion of the concave surface of the shock wave transducer 21. Reference numeral 23 indicates a water bag.

A pulser (PLS) 24 is employed to transmit a pulsatory excitation signal to the shock wave transducer 21. An ultrasonic transmitting/receiving circuit (UL-TR/RV) 25 is connected to the ultrasonic probe 22 so as to send another pulse signal thereto for a sector scanning excitation. Thus, the transmitting/receiving circuit 25 transmits the ultrasonic wave beam to the biological body under medical examination and receives an echo pulse therefrom. A signal processing circuit (SIG-PRC) 26 is connected to the transmitting/receiving circuit 25 so as to amplitude-detect an output signal therefrom. A signal converting circuit (SIG-COV) 27 is connected to the signal processing circuit 26, whereby an output signal from the signal processing circuit 26 is converted into a corresponding video signal. This video signal is supplied to a display unit 28, e.g., a television monitor so that a sector (B-mode)tomographic image 29 is displayed on the display unit 28. A probe position detecting unit (PRB-POS DET) 30 is further employed to detect a dimension (distance) of the ultrasonic probe 22 along a depth direction thereof, inserted into the ultrasonic applicator 20. A focal point calculation unit (F-POS CAL) 31 is connected to the probe position detecting unit 30 in order to calculate a focal point of a shock wave from a shock wave generating source, i.e., the ultrasonic shock wave transducer 21, on the monitor screen of the display unit 28. The calculated focal point from the focal point calculating unit 31 is displayed as a focal point marker 32 at a corresponding marker position on the monitor screen.

A probe rotation angle detecting unit (PRB-$\theta$ DET) 33 is further employed so as to detect a present tomographic (slice) plane direction of the ultrasonic probe 22, namely a rotation angle of the ultrasonic probe 22. To make a coincidence between a position of the shock wave generating source and an article to be disintegrated, an applicator moving mechanism (UL-APPL TRNS) 34 for mechanically moving the ultrasonic applicator 20 in various directions (will be described in detail). This applicator moving mechanism 34 is controlled by a transport mechanism control unit 35, so that the ultrasonic applicator 20 is transported along various directions X, Y, Z in a three-dimensional coordinate system, in an $\alpha$ (alpha) direction (i.e., a rotation direction around the X axis), and a $\beta$ (beta) direction (i.e., a rotation direction around the Y axis).

Reference numeral 36 indicates a transport control mode selecting switch (MOV-MOD SELC) 36 for selecting a transport control mode of the ultrasonic applicator 20 between an absolute coordinate system transport control mode and a probe slice plane (probe movement coordinate system) transport control mode. When the probe slice plane transport control mode is selected by operating this selecting switch 36, transport control information on the probe slice plane must be converted into transport information on the absolute coordinate system. To this end, a probe slice plane/absolute coordinate transport direction converting unit (PRB-SLC/ABS-MOV CONVT) 37 is employed and connected to the transport mode selecting switch 36 and probe rotation angle detecting unit 33. An operation panel (MANI-PAN) 38 on which three joy sticks are mounted is employed so as to input various transport control information into the transport mechanism control unit 35. It should be noted that the transport control mode which has been selected by the selecting switch 36 is displayed on the monitor screen of the display unit 28.

## COORDINATE SYSTEM CONVERTING METHOD

A description will now be made of a major feature of the present invention, i.e., a converting method for performing a conversion between a probe slice plane transport control mode and an absolute coordinate system transport control mode.

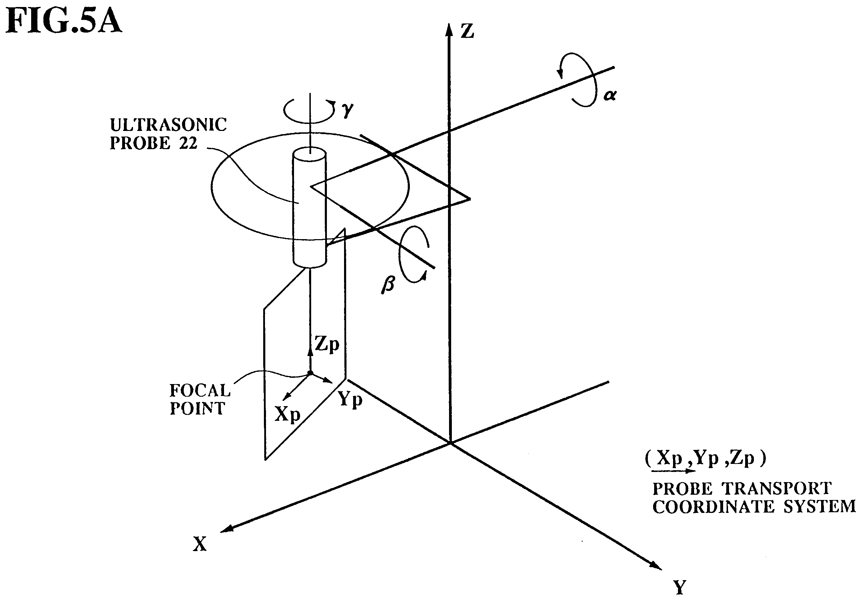

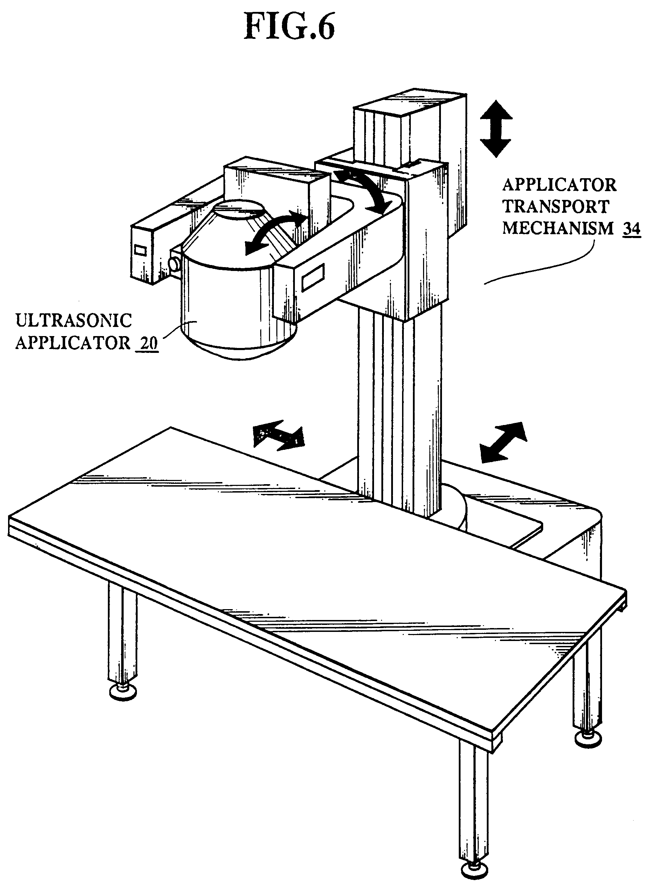

In Fig. 5A, there is shown an illustration of a relationship between the absolute coordinate system (X, Y, Z) and the probe movement coordinate system (Xp, Yp, Zp). As apparent from Fig. 5A, the probe movement coordinate system corresponds to such a coordinate system which is rotated around the X, Y, and Z axes by $\alpha$, $\beta$, and $\gamma$ directions. The actual moving directions of the ultrasonic applicator 20 are illustrated by arrows in Fig. 6.

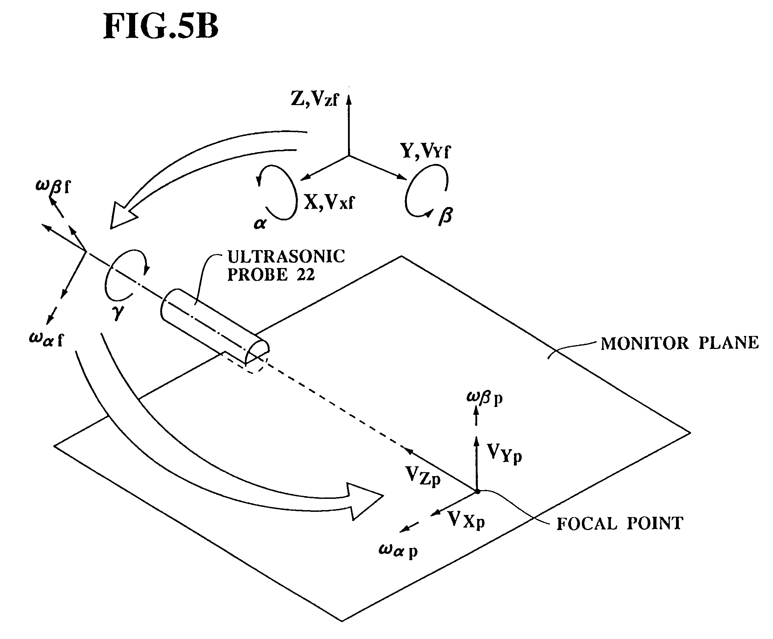

Furthermore, Fig. 5B illustrates the coordinate system conversion when the probe transport control mode is selected.

A detailed coordinate system conversion will be described later.

## OVERALL OPERATION

Referring now to Figs. 4 through 5, an overall operation of the first shock wave generating apparatus 100 will be described.

As previously stated, the ultrasonic applicator 20 is mainly constructed of the probe position detecting unit 30 for detecting the positional relationship between this applicator 20 and ultrasonic probe 22, and also the probe rotation angle detecting unit 33 for detecting the probe rotation angle with respect to the ultrasonic applicator 20. The positional signal derived from the probe position detecting unit 30 is supplied to the focal point calculating unit 31 in which the focal point of the ultrasonic shock wave is calculated with respect to the monitor screen of the display unit 28. The resultant focal point is displayed as the focal point marker 32 on this monitor screen.

## FOCUSING ARTICLE TO BE DISINTEGRATED

To focus the ultrasonic shock wave onto an article to be disintegrated (i.e., an image 40), the applicator transport control mechanism 34 controls the transportations of the ultrasonic applicator 20 in accordance with the following transport control modes. As previously described, there are introduced two transport control modes for this applicator 20. As the first transport control mode, the applicator 20 is transported in parallel to, or rotated around the absolute coordinate system which is determined by the actual construction of the first shock wave generating apparatus 100, as represented in Figs. 5A and 6. Further, as the second transport control mode, assuming now that the slice plane of the ultrasonic probe 22, i.e., scanning direction is used as a reference, the applicator 20 is transported in parallel to, or rotated around an axial direction positioned parallel to or perpendicular to this reference plane, as represented in Fig. 5B.

These two transport control modes may be selected by operating the transport control mode selecting switch 36. In the first preferred embodiment, when the probe slice plane transport control mode is selected, this selected control mode is displayed on the monitor screen of the display unit 28. Then, various instructions, e.g., X, Y, Z, $\alpha$ and $\beta$ given by the joy sticks of the operation panel 38 are once supplied to the probe slice plane/absolute coordinate transport direction converting unit 37. As a result, based upon the probe rotation angle information derived from the probe rotation angle detecting unit 33, the above-described probe slice plane transport control data are converted into the absolute coordinate system transport control data which will be then furnished to the transport mechanism control unit 35.

## COORDINATE SYSTEM CONVERSION

The coordinate system conversion between the probe movement coordinate system determined by the ultrasonic tomographic image (slice image) and the absolute coordinate system determined by the construction of the shock wave generating apparatus 100, is another major feature of the present invention. Then, one of the typical coordinate system conversion methods according to the present invention will now be described in detail.

In the absolute transport control mode selected by the transport control mode selecting switch 36 shown in Fig. 4, the various operation instructions given by the joy sticks 9A to 9C are directly applied via switch contacts 36A and 36B of the selecting switch 36 to the transport mechanism control unit 35. As a result, the ultrasonic applicator 20, i.e., ultrasonic probe 22 and shock wave generating source 21 is moved along the 3-dimensional axes X, Y and Z of the absolute coordinate system by the applicator transport mechanism 34 under the control of the transport mechanism control unit 35.

To the contrary, when the probe slice plane transport control mode is selected by the selecting switch 36, the various operation instructions within the probe slice plane given by the joy sticks 9A to 9C are once converted into the transport control data for the absolute coordinate system by the probe slice plane/absolute coordinate transport direction converting unit 37 and thereafter the resultant transport control data are supplied via the contact 36A to the transport mechanism control unit 35. Then, similarly the ultrasonic applicator 20 is moved along the three-dimensional axes X, Y, and Z of the absolute coordinate system by the applicator transport mechanism 34 under the control of the transport mechanism control unit 35. It should be noted that three-dimensional axes Xp, Yp, Zp are defined by the probe transport coordinate system.

In the first preferred embodiment, since there is such a positional relationship as to the movement of the ultrasonic applicator 20, as represented in Fig. 5A, the transport instruction signals given by the joy sticks 9A to 9C are converted by the probe slice plane/absolute coordinate transport direction converting unit 37.

The following operation velocity instructions are inputted by the joy sticks: $V_X$, $V_Y$, $V_Z$, $\omega_\alpha$ and $\omega_\beta$.

When the absolute coordinate transport control mode is selected,

$$\begin{pmatrix} V_{Xf} \\ V_{Yf} \\ V_{Zf} \\ \omega_{\alpha f} \\ \omega_{\beta f} \end{pmatrix} = \begin{pmatrix} V_{Xf} \\ V_{Yf} \\ V_{Zf} \\ \omega_{\alpha f} \\ \omega_{\beta f} \end{pmatrix} \qquad (1)$$

Note that a superscript "f" indicates a gradient of a relevant joy stick which has been inputted in the absolute coordinate transport control mode.

When the probe slice plane transport control mode is selected, the following coordinate system conversion is carried out by utilizing the illustration shown in Fig. 5B:

$$\begin{pmatrix} V_{Xf} \\ V_{Yf} \\ V_{Zf} \end{pmatrix} = \begin{pmatrix} \cos\gamma & -\sin\alpha & 0 \\ \sin\gamma & \cos\gamma & 0 \\ 0 & 0 & 1 \end{pmatrix} \begin{pmatrix} \cos\beta & 0 & \sin\beta \\ 0 & 1 & 0 \\ -\sin\beta & 0 & \cos\beta \end{pmatrix} \begin{pmatrix} 1 & 0 & 0 \\ 0 & \cos\alpha & -\sin\alpha \\ 0 & \sin\alpha & \cos\alpha \end{pmatrix} \begin{pmatrix} V_{Xp} \\ V_{Yp} \\ V_{Zp} \end{pmatrix} \qquad (2)$$

$$\begin{pmatrix} \omega_{\alpha f} \\ \omega_{\alpha f} \end{pmatrix} = \begin{pmatrix} \cos\gamma & -\sin\gamma \\ \sin\gamma & \cos\gamma \end{pmatrix} \begin{pmatrix} \omega_{\alpha p} \\ \omega_{\beta p} \end{pmatrix}$$

Note that a superscript "p" denotes a gradient of a relevant joy stick which has been inputted in the probe slice plane transport control mode.

Matrixes "$P_f$" and "$P_p$" to execute these coordinate conversions are so-called:

$P_f$ - - - (absolute coordinate transport)

$P_p$ - - - (probe slice plane transport)

In the first shock wave generating apparatus 100 shown in Fig. 4, when the probe slice plane transport control mode is selected by operating the transport control mode selecting switch 36, the operation instruction velocities given by the corresponding joy sticks 9A to 9C are converted into the required absolute coordinate transport control mode data by utilizing the above-described matrixes $P_f$ and $P_p$, which are then supplied to the transport mechanism control unit 35.

PRACTICAL CIRCUIT ARRANGEMENT OF MAJOR ARRANGEMENT OF FIRST SHOCK WAVE GENERATING APPARATUS

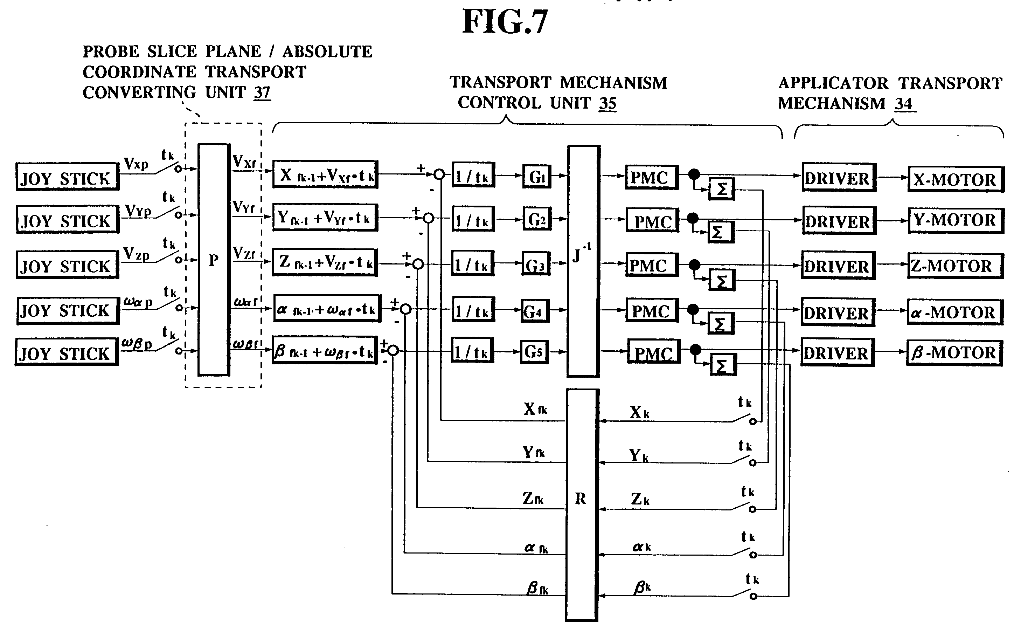

In Fig. 7, there is shown a practical circuit arrangement of the applicator transport mechanism 34, transport mechanism control unit 35, and probe slice plane/absolute coordinate transport converting unit 37 functioning as the major featured arrangement of the first shock wave generating apparatus 100 shown in Fig. 4.

This practical circuit arrangement will now be summarized. The joy stick 9 inputs the various operation instructions, i.e., velocities of $V_{Xp}$, $V_{Yp}$, and $V_{Zp}$, and rotation angles of $\omega_{\alpha p}$ and $\omega_{\beta p}$ into the probe slice plane/absolute coordinate transport converting unit 37 in which the above-described converting matrixes $P_f$ and $P_p$ are calculated. The converted absolute coordinate transport control data of $V_{Xf}$, $V_{Yf}$, $V_{Zf}$, $\omega_{\alpha f}$ and $\omega_{\beta f}$ are furnished to the transport mechanism control unit 35 which is constructed of, for instance, gain controllers $G_1$ to $G_5$, pulse motor controllers , and counters $\Sigma$. The

applicator transport controlling data from the transport mechanism control unit 35 are supplied to the applicator transport mechanism 34 which is constructed of drivers, and X, Y, Z-axis motors and also $\alpha$, $\beta$-axis motors.

As apparent from the circuit arrangement of Fig. 7, a feedback path is constructed in the transport mechanism control unit 37 in order to reduce a difference occurring between the transport instructions given by the joy sticks and the actual transport.

A symbol "$J^{-1}$" of the transport mechanism control unit 35 indicates Jacobian's inverse matrix. This inverse matrix defers with each other depending upon the absolute transport control mode and probe slice plane transport control mode, and therefore is expressed by the following two matrixes, i.e., "$J^{-1}_f$" and "$J^{-1}_p$".

$$J^{-1}_f = \begin{pmatrix} 10000 \\ 01000 \\ 00100 \\ 00010 \\ 00001 \end{pmatrix} = E \text{ (unit matrix), and}$$

$$J^{-1}_p = \begin{bmatrix} 1 & 0 & 0 & 0 & Z_f\cos\beta \\ 0 & 1 & 0 & -Z_f\cos\alpha\cos\beta & Z_f\sin\alpha\sin\beta \\ 0 & 0 & 1 & -Z_f\sin\alpha\cos\beta & -Z_f\cos\alpha\sin\beta \\ 0 & 0 & 0 & 1 & 0 \\ 0 & 0 & 0 & 0 & 1 \end{bmatrix}$$

Furthermore, A symbol "R" of the transport mechanism control unit 35 represents such a calculation. That is, the present position of the ultrasonic applicator 20 is calculated from the motor transporting amounts of the respective axes, and then is given by the following formula:

$$\begin{pmatrix} X_f \\ Y_f \\ Z_f \\ \alpha_f \\ \beta_f \end{pmatrix} = \begin{pmatrix} X-Z_f\sin\beta \\ Y+Z_f\sin\alpha\cos\beta \\ Z-Z_f\cos\alpha\cos\beta \\ \alpha \\ \beta \end{pmatrix}$$

It should be noted that this present position calculation is commonly utilized for both transport control modes.

Referring back to the circuit arrangement to Fig. 4, the applicator transport mechanism 35 is operated in the absolute transport control mode during the actual operation, taking account of body attitudes of a patient (biological body) and positions of various organs. As a result, an image 40 of the article to be disintegrated is displayed in the tomographic (slice) image 29 of the patient on the display unit 28. Subsequently, this absolute coordinate transport control mode is changed into the probe slice plane transport control mode by operating the transport control mode selecting switch 36 so as to make both the image 40 of the article to be disintegrated and the focal point marker 32 of the shock wave generating source 21 coincident. Thereafter, even when an operator manipulates the operation panel 38 so as to input various movement data, e.g., X, Z, $\alpha$ for the transportation of the ultrasonic applicator 20, the image 40 of the article to be disintegrated does not disappear from the monitor screen of the display unit 28, because the movements of the applicator 20 are controlled by the transport mechanism control unit 35 into which these input movement data have been converted into the probe slice plane transport control data and then are supplied. Moreover, even if the image 40 of the article to be disintegrated disappears due to some accidental phenomena, for instance, a movement of a patient, the transport control data Y and $\beta$ are supplied to be converting unit 37 so that the applicator 20 may be transported in a direction perpendicular to the slice plane so as to again display this image 40 on the monitor screen, since the article to be disintegrated most probably exists in slice planes adjacent to the above-described slice plane from which the image 40 of the article to be disintegrated has just disappeared.

As apparent from the foregoings, the present invention is not limited to the above-described first preferred embodiment, but may be modified without departing from the technical scope of the present invention. For instance, the probe ratation angle detecting unit may be realized by combining a gear and a potentiometer.

As previously described in detail, in accordance with the shock wave generating apparatus of the present invention, since when the image of the article to be disintegrated is displayed in the tomographic image produced by the ultrasonic probe 22, this condition is sensed and thereafter both the shock wave generating source and ultrasonic probe are transported along the slice plane (tomographic image), the image of the article to be disintegrated can be continuously displayed within the slice plane while moving the shock wave generating source and ultrasonic probe. In other words, since the moving direction of the applicator can be automatically determined by the probe slice plane/absolute coordinate transport direction converting unit, the article's image never disappears from the display screen. As a consequence, the identifying operations for positioning the article to be disintegrated can be very simply and readily performed.

**Claims**

1. A shock wave generating apparatus comprising:

source means (21) for producing a shock wave and transmitting said shock wave to a biological body under medical examination;

ultrasonic imaging means (200) including ultrasonic probe means (22) to project an ultrasonic wave beam to said biological body for producing and displaying an ultrasonic tomographic image of tissue within said biological body;

probe position denting means (30) for detecting a position of said ultrasonic probe means (22) so as to produce a positioning signal when an image of an article to be disintegrated located within said tissue of said biological body is being displayed in said ultrasonic tomographic image;

transporting means (34) for changing the relative position between said article to be disintegrated and said source means which transporting means (34) transports both said source means (21) and said ultrasonic probe means (200);

transport controlling means (35, 36, 37, 38) for controlling the transport means in response to the position signal as to position the article to be disintegrated to coincide with the focal point of said shock wave produced by said source means,

and generating means (31) provided for generating a marker in said ultrasonic tomographic image indicative of the focal point of said shock wave produced by said source means (21),

characterized in that

said probe position detecting means (30) detects a slice plane position of said ultrasonic probe means (22), the axis of which is adapted to rotate so that the ultrasonic tomographic image can be viewed along a rotable slice plane; and

the transporting controlling means (35, 36, 37, 38) controls the transport means (34) so as to transport both said source means (21) and said ultrasonic probe means (22) along the detected slice plane in response to the slice plane positioning signal, whereby said marker is coincident with said image of said article to be disintegrated.

2. A shock wave generating apparatus as claimed in claim 1, wherein said transport controlling means (35, 36, 37, 38) includes:

a transport control mode instruction unit (36, 38) for instructing one of an absolute coordinate transport control mode and an ultrasonic slice plane transport control mode and

a transport mode converting unit (37) for converting said ultrasonic slice plane transport control mode into a corresponding converted absolute coordinate transport control mode when said ultrasonic slice plane transport control mode is selected by a transport control mode selecting switch,

whereby said transport controlling means is controlling said transporting means (34) in response to one of said absolute coordinate transport control mode and said converted absolute coordinate transport control mode, whereby said marker is made coincident with said image of said article to be disintegrated, even when said ultrasonic slice plane transport control mode is instructed.

3. A shock wave generating apparatus as claimed in claim 2, wherein said transport control mode instruction unit (36, 38) is constructed of an operation panel (38) having a joystick (9) and the transport control mode selecting switch (36).

4. A shock wave generating apparatus (100) as claimed in Claim 2, wherein said transport mechanism controlling unit (35) controls said transporting means (34) so as to transport both the shock wave generating source means (21) and ultrasonic probe means (22) along a three-dimensional coordinate system (X, Y, Z) defining at least said absolute coordinate system.

5. A shock wave generating apparatus (100) as claimed in Claim 2, wherein said transport mode converting unit (37) converts said ultrasonic slice plane transport control mode into said corresponding converted absolute coordinate transport control mode by utilizing a predetermined matrix ($P_f$, $P_p$).

6. A shock wave generating apparatus (100) as claimed in Claim 2, further comprising:
   a probe rotation angle detector (33) for detecting a rotation angle of said probe means (22) to produce actual rotation angle data.

7. A shock wave generating apparatus (100) as claimed in Claim 6, wherein said transport control mode instruction unit (38:36) inputs velocity data and desirable rotation angle data on said transporting means (34) into said transport mode converting unit (37), whereby said coordinate conversion is performed therein based upon said inputted velocity data and desirable rotation angle data with reference to said actual rotation angle data.

8. A shock wave generating apparatus (100) as claimed in Claim 1, wherein said transporting means (34) including three-dimensional axes motors and two rotation-angle motors.

9. A shock wave generating apparatus (100) as claimed in Claim 1, wherein said source means (21) is constructed of an ultrasonic transducer (21).

10. A shock wave generating apparatus (100) as claimed in Claim 1, wherein said ultrasonic probe means includes:
   an ultrasonic probe (22) for projecting the ultrasonic wave beam to the biological body; and,
   a probe position detector (30) for detecting a relative position between said ultrasonic probe (22) and said shock wave source means (21) so as to produce said positional condition signal of said ultrasonic probe means (22).

**Patentansprüche**

1. Gerät zum Erzeugen von Stoßwellen, enthaltend:
   eine Quellenvorrichtung (21) zum Erzeugen einer Stoßwelle und zum Übertragen der Stoßwelle auf einen medizinisch untersuchten biologischen Körper;
   eine Ultraschallbildvorrichtung (200) mit einer Ultraschallsondenvorrichtung (22) zum Aussenden eines Ultraschallwellenstrahl auf den biologischen Körper, um ein tomographisches Ultraschallbild von Gewebe innerhalb des biologischen Körpers zu erzeugen und anzuzeigen;
   eine Sondenpositionsdetektorvorrichtung (30) zum Erfassen einer Position der Ultraschallsondenvorrichtung (22), um ein Positionssignal zu erzeugen, wenn ein Bild eines innerhalb des Gewebes des biologischen Körpers angeordneten, zu zerkleinernden Gegenstandes in dem tomographischen Ultraschallbild angezeigt wird;
   eine Transportvorrichtung (34) zum Verändern der relativen Position zwischen dem zu zerkleinernden Gegenstand und der Quellenvorrichtung, welche Transportvorrichtung (34) sowohl die Quellenvorrichtung (21) als auch die Ultraschallsondenvorrichtung (22) transportiert;
   eine Transportsteuervorrichtung (35, 36, 37, 38) zum Steuern der Transportvorrichtung ansprechend auf das Positionssignal, derart, daß der zu zerkleinernde Gegenstand mit dem Brennpunkt der von der Quellenvorrichtung erzeugten Stoßwelle zusammenfällt, und
   eine Erzeugungsvorrichtung (31) zum Erzeugen einer Markierung in dem tomographischen Ultraschallbild, die den Brennpunkt der von der Quellenvorrichtung (21) erzeugten Stoßwelle anzeigt,
   dadurch **gekennzeichnet,** daß
   die Sondenpositionsdetektorvorrichtung (30) eine Schnittebenenposition der Ultraschallsondenvorrichtung (22) erfaßt, deren Achse derart drehbar ist, daß das tomographische Ultraschallbild längs einer drehbaren Schnittebene betrachtbar ist; und
   die Transportsteuervorrichtung (35, 36, 37, 38) die Transportvorrichtung (34) derart steuert, daß sowohl die Quellenvorrichtung (21) als auch die Ultraschallsondenvorrichtung (22) längs der erfaßten Schnittebene, ansprechend auf das Schnittebenenpositionssignal, transportiert werden, wodurch die Markierung mit dem Bild des zu zerkleinernden Gegenstandes zusammenfällt.

2. Gerät zur Erzeugung von Stoßwellen nach Anspruch 1, wobei die Transportsteuervorrichtung (35, 36, 37, 38) enthält:
   eine Transportsteuerartanweisungseinheit (36, 38) zum Anweisen einer Absolutkoordinatentransportsteuerart oder einer Ultraschallschnittebenentransportsteuerart und

eine Transportartumwandlungseinheit (37) zum Umwandeln der besagten Ultraschallschnittebenentransportsteuerart in eine entsprechende umgewandelte Absolutkoordinatentransportsteuerart, wenn die Ultraschallschnittebenentransportsteuerart mittels eines Transportsteuerartwählschalters gewählt ist,

wodurch die Transportsteuervorrichtung die Transportvorrichtung (34) ansprechend auf die Absolutkoordinatentransportsteuerart oder die umgewandelte Absolutkoordinatentransportsteuerart steuert, wodurch die Markierung mit dem Bild des zu zerkleinernden Gegenstandes zusammenfällt, selbst wenn die besagte Ultraschallschnittebenentransportsteuerart angewiesen ist.

3. Gerät zur Erzeugung von Stoßwellen nach Anspruch 2, wobei die Transportsteuerartanweisungseinheit (36, 38) aus einem Bedienfeld (38) mit einem Steuerknüppel (9) und dem Transportsteuerartwählschalter (36) aufgebaut ist.

4. Gerät (100) zum Erzeugen von Stoßwellen nach Anspruch 2, wobei die Transportmechanismussteuereinheit (35) die Transportvorrichtung (34) derart steuert, daß sowohl die Stoßwellenerzeugungsquellenvorrichtung (21) als auch die Ultraschallsondenvorrichtung (22) längs eines dreidimensionalen Koordinatensystems transportiert werden, das zumindest das besagte absolute Koordinatensystem definiert.

5. Gerät (100) zum Erzeugen von Stoßwellen nach Anspruch 2, wobei die Transportartumwandlungseinheit (37) die besagte Ultraschallschnittebenentransportsteuerart in eine entsprechende umgewandelte Absolutkoordinatentransportsteuerart unter Verwendung einer vorbestimmten Matrix ($P_f$, $P_p$) umwandelt.

6. Gerät (100) zum Erzeugen von Stoßwellen nach Anspruch 2, zusätzlich enthaltend:
einen Sondendrehwinkeldetektor (33) zum Erfassen eines Drehwinkels der Sondenvorrichtung (22), um aktuelle Drehwinkelarten zu erzeugen.

7. Gerät (100) zum Erzeugen von Stoßwellen nach Anspruch 6, wobei die Transportsteuerartanweisungseinheit (36; 38) Geschwindigkeitsdaten und erwünschte Drehwinkeldaten an der besagten Transportvorrichtung (34) in die besagte Transportartumwandlungseinheit (37) eingibt, wodurch die Koordinatenumwandlung darin, basierend auf den eingegebenen Geschwindigkeitsdaten und den gewünschten Drehwinkeldaten unter Bezugnahme auf die aktuellen Drehwinkeldaten, durchgeführt wird.

8. Gerät (100) zum Erzeugen von Stoßwellen nach Anspruch 1, wobei die Transportvorrichtung (34) dreidimensionale Achsenmotoren und zwei Drehwinkelmotoren enthält.

9. Gerät (100) zum Erzeugen von Stoßwellen nach Anspruch 1, wobei die Quellenvorrichtung (21) aus einem Ultraschallwandler (21) aufgebaut ist.

10. Gerät (100) zum Erzeugen von Stoßwellen nach Anspruch 1, wobei die Ultraschallsondenvorrichtung enthält:
eine Ultraschallsonde (22) zum Aussenden des Ultraschallwellenstrahls auf den biologischen Körper; und
einen Sondenpositionsdetektor (30) zum Erfassen einer relativen Position zwischen der Ultraschallsonde (22) und der Stoßwellenquellenvorrichtung (21), um das besagte Positionsbedingungssignal der Ultraschallsondenvorrichtung (22) zu erzeugen.

## Revendications

1. Appareil générateur d'ondes de choc comprenant :
un moyen formant source (21) pour produire une onde de choc et pour émettre ladite onde de choc vers un corps biologique sous examen médical ;
un moyen de formation (200) d'image par ultrasons incluant un moyen formant sonde à ultrasons (22) pour projeter un faisceau d'ondes ultrasonores vers ledit corps biologique pour produire et pour afficher une image tomographique à ultrasons du tissu à l'intérieur du corps biologique ;
un moyen de détection de position de sonde (30) pour détecter la position dudit moyen formant sonde à ultrasons (22) de façon à produire un signal de positionnement lorsque l'image d'un élément à désintégrer situé à l'intérieur dudit tissu dudit corps biologique est en cours d'affichage dans ladite image tomographique à ultrasons ;
un moyen de déplacement (34) pour modifier la position relative entre ledit élément à désintégrer et ledit moyen formant source, lequel moyen de déplacement (34) déplace à la fois ledit moyen formant source (21) et ledit moyen formant sonde à ultrasons (22) ;
un moyen de commande de déplacement (35, 36, 37, 38) pour commander le moyen de déplacement en réponse au signal de position pour positionner l'élément à désintégrer pour qu'il coïncide avec le point focal de ladite onde de choc produite par ledit moyen formant source ; et

un moyen générateur (31) prévu pour produire, dans ladite image tomographique à ultrasons, un marqueur indiquant le point focal de ladite onde de choc produite par ledit moyen formant source (21) ;

caractérisé :

en ce que ledit moyen de détection de position de sonde (30) détecte une position de plan de tranche dudit moyen formant sonde à ultrasons (22) dont l'axe est conçu pour tourner de façon telle que l'image tomographique à ultrasons puisse être visualisée suivant un plan de tranche tournant ; et

en ce que le moyen de commande de déplacement (35, 36, 37, 38) commande le moyen de déplacement (34) de façon à déplacer à la fois ledit moyen formant source (21) et ledit moyen formant sonde à ultrasons (22) dans le plan de tranche détecté, en réponse au signal de positionnement de plan de tranche, ce par quoi ledit marqueur coïncide avec ladite image dudit élément à désintégrer.

2. Appareil générateur d'ondes de choc selon la revendication 1, dans lequel ledit moyen de commande de déplacement (35, 36, 37, 38) comprend :

un module d'instructions de mode de commande de déplacement (36, 38) pour ordonner l'un d'un mode de commande de déplacement en coordonnées absolues et d'un mode de commande de déplacement dans le plan de tranche d'ultrasons ; et

un module de conversion de mode de déplacement (37) pour convertir ledit mode de commande de déplacement dans le plan de tranche d'ultrasons en mode de commande de déplacement en coordonnées absolues converties de manière correspondante, lorsque l'on choisit, par l'interrupteur de sélection de mode de commande de déplacement, ledit mode de commande de déplacement dans le plan de tranche d'ultrasons ;

ce par quoi ledit moyen de commande de déplacement commande ledit moyen de déplacement (34) en réponse à l'un dudit mode de commande de déplacement en coordonnées absolues et dudit mode de commande de déplacement en coordonnées absolues converties, ledit marqueur étant ainsi mis en coïncidence avec ladite image dudit élément à désintégrer, même si l'on ordonne le mode de commande de déplacement dans le plan de tranche d'ultrasons.

3. Appareil générateur d'ondes de choc selon la revendication 2, dans lequel ledit module d'instructions de mode de commande de déplacement (36, 38) est constitué d'un tableau de manoeuvre (38) comportant une manette (9) et de l'interrupteur de sélection de mode de commande de déplacement (36).

4. Appareil générateur d'ondes de choc (100) selon la revendication 2, dans lequel ledit module de commande de mécanisme de déplacement (35) commande ledit moyen de déplacement (34) de façon à déplacer à la fois le moyen formant source génératrice d'ondes de choc (21) et le moyen formant sonde à ultrasons (22) suivant un système de coordonnées tridimensionnelles (X, Y, Z) définissant au moins ledit système de coordonnées absolues.

5. Appareil générateur d'ondes de choc (100) selon la revendication 2, dans lequel ledit module de conversion de mode de déplacement (37) convertit ledit mode de commande de déplacement dans le plan de tranche d'ultrasons en ledit mode de commande de déplacement en coordonnées absolues converties correspondantes, par l'utilisation d'une matrice prédéterminée ($P_f$, $P_p$).

6. Appareil générateur d'ondes de choc (100) selon la revendication 2, comprenant en outre :

un détecteur d'angle de rotation de sonde (33) pour détecter l'angle de rotation dudit moyen formant sonde (22) pour produire des données d'angle de rotation réelles.

7. Appareil générateur d'ondes de choc (100) selon la revendication 6, dans lequel ledit module d'instructions de mode de commande de déplacement (38 ; 36) entre des données de vitesse et des données d'angle de rotation, souhaitables pour ledit moyen de déplacement (34), dans ledit module de conversion de mode de déplacement (37), en y effectuant ainsi ladite conversion de coordonnées en se basant sur lesdites données de vitesse et lesdites données d'angle de rotation souhaitables entrées, en se référant auxdites données d'angle de rotation réelles.

8. Appareil générateur d'ondes de choc (100) selon la revendication 1, dans lequel ledit moyen de déplacement (34) comprend des moteurs d'axes tridimensionnels et deux moteurs d'angle de rotation.

9. Appareil générateur d'ondes de choc (100) selon la revendication 1, dans lequel ledit moyen formant source (21) est constitué d'un transducteur à ultrasons (21).

10. Appareil générateur d'ondes de choc (100) selon la revendication 1, dans lequel ledit moyen formant sonde à ultrasons comprend :

une sonde à ultrasons (22) pour projeter le faisceau d'ondes ultrasonores sur le corps biologique ; et

un détecteur de position de sonde (30) pour détecter la position relative entre ladite sonde à ultrasons (22) et ledit moyen formant source d'ondes de choc (21) de façon à produire ledit signal d'état de position dudit moyen formant sonde à ultrasons (22).

EP 0 397 056 B1

PRIOR ART
# FIG.1

PRIOR ART
# FIG.2

PRIOR ART
# FIG.3A

PRIOR ART
# FIG.3B

PRIOR ART
# FIG.3C

PRIOR ART
# FIG.3D

FIG.4

SHOCK WAVE GENERATING APPARATUS 100

ULTRASONIC IMAGNG MEANS 200

PLS — 24
PRB-θ DET — 33
UL-APPL TRNS — 34
TRNS CONT — 35
PRB-SLC / ABS-MOV CONVT — 37
MANI-PAN — 38
MOV-MOD SELC — 36
36A, 36B
9A, 9B, 9C
X, Y, Z α, β

21, 22, 23, 20
PRB-POS DET — 30
F-POS CAL — 31
UL-TR/RV — 25
SIG-PRC — 26
SIG-COV — 27
28, 29, 32, 40

# FIG.5A

ULTRASONIC
PROBE 22

FOCAL
POINT

$( Xp, Yp, Zp )$

PROBE TRANSPORT
COORDINATE SYSTEM

EP 0 397 056 B1

# FIG.5B

EP 0 397 056 B1

# FIG.6

APPLICATOR
TRANSPORT
MECHANISM 34

ULTRASONIC
APPLICATOR 20

# FIG.7

PROBE SLICE PLANE / ABSOLUTE
COORDINATE TRANSPORT
CONVERTING UNIT 37

TRANSPORT MECHANISM
CONTROL UNIT 35

APPLICATOR TRANSPORT
MECHANISM 34

EP 0 397 056 B1